# EUROPEAN PATENT SPECIFICATION

(11) **EP 4 490 549 B1**
(45) Date of publication and mention of the grant of the patent: **18.11.2026**
(21) Application number: 23709673.0
(22) Date of filing: 06.03.2023
(51) Int. Cl.: G01T 1/29

(54) **METHOD FOR DETERMINING RELATIVE DETECTOR ELEMENT EFFICIENCIES IN A PET-SCANNING DEVICE**
VERFAHREN ZUR BESTIMMUNG DER RELATIVEN EFFIZIENZ VON DETEKTORELEMENTEN IN EINER PET-ABTASTVORRICHTUNG
PROCÉDÉ DE DÉTERMINATION DES EFFICACITÉS RELATIVES DES ÉLÉMENTS DÉTECTEURS DANS UN DISPOSITIF DE BALAYAGE PET

(30) Priority: 10.03.2022 EP 22161285
(43) Date of publication of application: 15.01.2025
(73) Proprietor: Positrigo AG, 8005 Zürich (CH)
(72) Inventor: MIKHAYLOVA, Ekaterina, 5223 Riniken (CH); JEHL, Markus, 8045 Zürich (CH)
(74) Representative: Rutz, Andrea
(86) International application number: PCT/EP2023/055555
(87) International publication number: WO 2023/169978

(56) References cited:
- WO-A1-2020/015384
- US-A1- 2015 297 168
- US-B2- 10 101 474
- US-B2- 6 980 683
- ANDRÉ SALOMON ET AL: "A Self-Normalization Reconstruction Technique for PET Scans Using the Positron Emission Data", IEEE TRANSACTIONS ON MEDICAL IMAGING, IEEE, USA, vol. 31, no. 12, 1 December 2012 (2012-12-01), pages 2234 - 2240, XP011474011, ISSN: 0278-0062, DOI: 10.1109/TMI.2012.2213827
- JONATHAN K POON ET AL: "A simulation study of a long axial field of view whole-body PET scanner using cylindrical and anthropomorphic phantoms", NUCLEAR SCIENCE SYMPOSIUM CONFERENCE RECORD, 2008. NSS '08. IEEE (19-25 OCT. 2008), IEEE, PISCATAWAY, NJ, USA, 19 October 2008 (2008-10-19), pages 4999 - 5006, XP031418632, ISBN: 978-1-4244-2714-7
- MCELROY D P ET AL: "Characterization and Readout of MADPET-II Detector Modules: Validation of a Unique Design Concept for High Resolution Small Animal PET", IEEE TRANSACTIONS ON NUCLEAR SCIENCE, IEEE, USA, vol. 52, no. 1, 1 February 2005 (2005-02-01), pages 199 - 204, XP011129940, ISSN: 0018-9499, DOI: 10.1109/TNS.2004.843114

## Description

### TECHNICAL FIELD

The present invention relates to a method for determining relative detector element efficiencies in a positron emission tomography (PET)-scanning device. The method can particularly be used for the calibration of a PET-scanning device and/or for the correction of PET-data acquired by the PET-scanning device.

### PRIOR ART

In positron emission tomography (PET)-imaging, in order to obtain images of high quality and with a minimum of artifacts, it is necessary to carry out a number or corrections before, within and/or after the reconstruction procedure. These particularly include normalization of detector efficiencies, in order to cancel out the variations in the sensitivities and in the geometric factors of different detector crystals.

In the state of the art, three normalization techniques are known for use on clinical or preclinical PET-scanners: i. direct normalization, ii. component-based normalization, and iii. self-normalization. In the case of the direct method, the normalization coefficient for each line of response (LOR) is inversely proportional to the number of counts acquired by scanning a homogeneous and well-centered cylindrical phantom for several hours. Component-based normalization is a well-established approach applied in small-animal PET-scanners owing to sufficient statistical accuracy. Component-based normalization is based on determining and calculating factors affecting LOR sensitivity, such as intrinsic detector efficiencies, blockrelated parameters, geometrical factors, and time and spatial misalignments separately. Normalization factors can be calculated using a rotating source, a uniform plane source, as well as a uniform cylindrical phantom. Both direct normalization and component-based normalization have the disadvantage that the situation between the phantom scan and the patient scan can change and that, therefore, the applied normalization of detector efficiencies might not be optimal anymore for the patient scan. Self-normalization is another option where the normalization coefficients are extracted solely from the PET emission data without the need for extra normalization measurements.

A method for PET detector efficiency normalization which requires the acquisition of sinogram data of a first and a second phantom is disclosed by US 7,718,954 B2.

US 9,693,751 B2 relates to a radiation therapy device and proposes a calibration of a PET detector of such a device by means of irradiating the patient with a defined radiation dose, in order to measure projection data of the activity in the patient's body caused by this radiation dose.

US 9,804,275 B2 discloses a method for determining crystal efficiency correction factors based directly on a scan of a patient. Sinogram-data of the patient that reflect the detected line of responses are used for this purpose.

In the method disclosed by US 10,482,596 B2, crystal efficiency normalization coefficients are determined based on patient data that is acquired during axial movement of the patient.

US 2004/0206897 A1 discloses a normalization method which is based on the positioning of a scatter source at the center of the scanner's field-of-view (FOV) and of a scatter-free source at a radial edge of the FOV.

WO 2014/209972 A1 discloses a method for determining normalization correction factors for a PET-scanner detector, which is based on the measurement of double coincidence events and/or on multiple detection coincidence events.

Further documents that relate to detector normalization methods for PET-scanners are US 7,038,212 B2, US 9,474,501 B2 and US 8,110,805 B2.

In the journal article Salomon A, Goldschmidt B, Botnar R, Kiessling F, Schulz V. A self-normalization reconstruction technique for PET scans using the positron emission data. IEEE Trans Med Imaging. 2012 Dec;31(12):2234-40. doi: 10.1109/TMI.2012.2213827. Epub 2012 Aug 17. PMID: 22910096*,* an iterative approach is proposed for determining relative detector efficiencies in a PET-scanner. For this purpose, both coincidences and singles emission data of a PET-scan are used.

In Ishikawa, A. & Kitamura, Keishi & Mizuta, T. & Tanaka, K. & Amano, M.. (2004). Self normalization for continuous 3D whole body emission data in 3D PET. Filtration & Separation - FILTR SEP. 6. 3634 - 3637 Vol. 6. 10.1109/NSSMIC.2004.1466670*,* a self-normalization approach is proposed in which a histogram of coincidence data is acquired, from which low-frequency distortions are removed. In order to obtain enough counts and correct results, the coincidence data need to be acquired and summed during continuous axial bed movement.

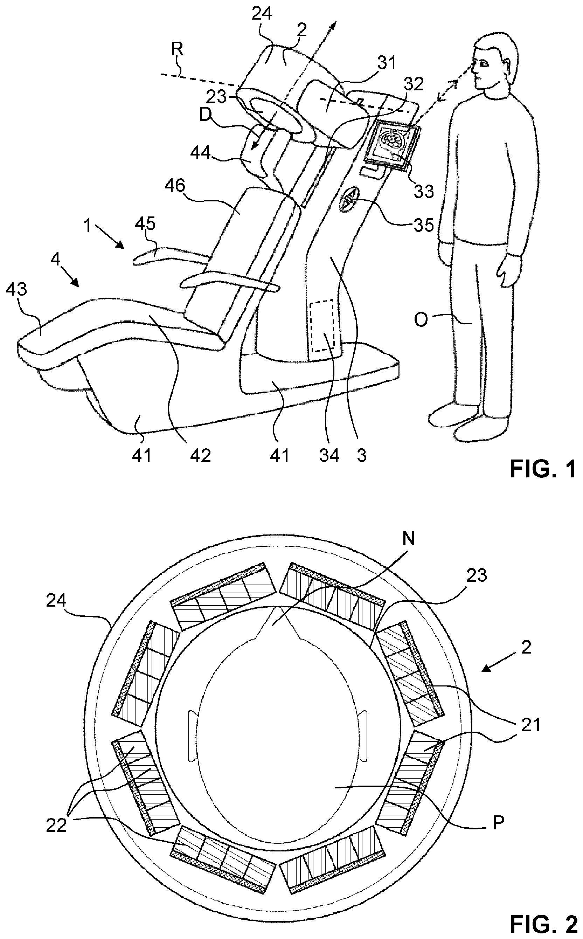

With the state-of-the-art methods as mentioned above, complicated and, thus, time-consuming mathematical calculations are required in some cases. In other cases, a long time duration and/or movement of the patient bed is necessary, in order to acquire sufficient data for determining the normalization factors. In almost all cases, the calculation of the normalization factors is adversely affected by geometric factors due to scanner design and irregular source distributions, which can hardly be avoided in practice as it is explained in more detail further below with respect to figures 2 to 8c.

In summary, the usual PET detector normalization methods according to the state of the art are inherently affected by geometric factors that are unavoidable in practice. Other methods that might overcome these drawbacks are known, but are computationally intensive and/or require extensive scan times.

### SUMMARY OF THE INVENTION

It is an object of the present invention to provide a method for determining relative detector element efficiencies in a positron emission tomography (PET)-scanning device in a particularly fast and reliable manner.

This object is solved by a method for determining relative detector element efficiencies in a PET-scanning device as claimed in claim 1. Further embodiments of the method are provided in dependent claims 2 to 10. A PET-scanning device which is configured to carry out a method for determining relative detector element efficiencies is claimed in claim 11. A computer program which serves for determining relative detector element efficiencies of a PET-scanning device is claimed in claim 12.

The present invention thus provides a method for determining relative detector element efficiencies in a PET-scanning device, comprising the steps of:
a) acquiring a histogram of singles emission data by means of a detector of the PET-scanning device, wherein each bin of the histogram is associated to one of a plurality of detector elements of the detector and reflects the sum of singles emissions detected by the respective detector element during a certain period of time; and
b) applying a spatial high-pass filter to the acquired histogram of singles emission data.

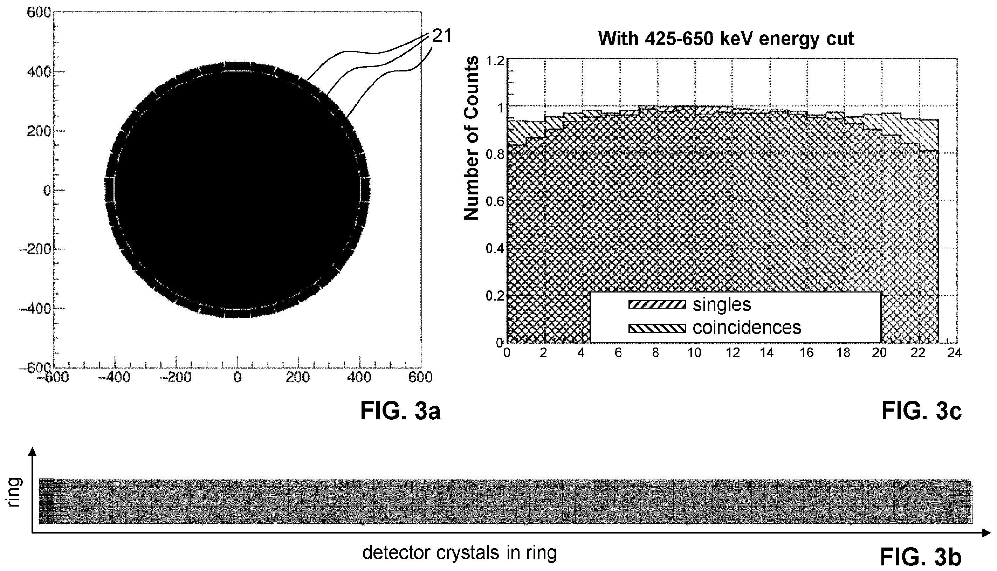

By acquiring a histogram of singles emission data and applying a spatial high-pass filter to the histogram data, the relative detector element efficiencies of the PET-scanning device can be determined in an accurate and particularly fast manner. It has surprisingly turned out that by using singles emission data instead of coincidence data, relative detector element efficiencies can be obtained that are much less adversely affected by geometric factors: With coincidence emission data, each data point of the histogram is always the result of a combined measurement of two detector elements. As a result, the measured data point is not only dependent on the actual distribution of the radiating source within the detector, but also on the geometric arrangement of the two detector elements with respect to the source. In contrast, the measurement of the data point in the histogram is much less affected by geometric factors of the detector, if singles emission data are considered, as is explained in more detail further down with respect to figures 3a to 6c. Furthermore, it was found that by using singles emission data instead of coincidence emission data, the acquisition time for obtaining the relative detector element efficiencies can be reduced significantly, because in this case all detected emission can be used for the histogram, while with coincidence emission data, a large part of the emissions detected by the detector elements do not constitute a coincidence detection and, therefore, need to be discarded.

By applying a spatial high-pass filter in step b), influences of the actual source distribution within the detector can be reduced considerably. Thus, by means of the spatial high-pass filter, it is possible to reduce influences related to non-optimal source distributions that adversely affect the determination of relative detector element efficiencies. Such a situation with a non-optimal source distribution is for example present, if the size of the radiating source is significantly smaller than the detector opening and/or if the source distribution is not symmetrical and/or not uniform. It was found that in these cases, by applying a spatial high-pass filter, the respective adverse influences to the determined relative detector element efficiencies can be greatly reduced.

As a result, by considering singles emission data instead of coincidences and by applying a spatial high-pass filter, it becomes possible to determine relative detector element efficiencies in a fast and accurate manner based on an almost arbitrary shape of a phantom or even directly on patient-data. Thus, in the former case, the obtained relative detector element efficiencies can be used for direct normalization, and in the latter case, the determined efficiencies can be used for self-normalization of detector efficiencies.

In the context of the present document, the term "singles emission data" or, in abbreviated form, "singles" refer to events in which a detector element detects a single photon emission. In contrast, the term "coincidence emission data" or, in abbreviated form, "coincidences" refer to simultaneous or coincident detections by two detector elements of a pair of photons moving in approximately opposite directions. Both terms "singles" and "coincidences" are well-known to the person skilled in the art in the field of PET-imaging.

The term "detector element" preferably refers to a single detector crystal of the detector of the PET-imaging device. A detector crystal is usually in the form of an inorganic scintillator crystal, which is used to record emitted photons, i.e. gamma-rays produced by the annihilation of positrons emitted by injected tracers. Thus, a detector element is preferably formed by the detector's smallest functional unit for detecting emitted photons. It is, however, also possible that each detector element is formed by a plurality of such detector crystals, which in this case are advantageously, but not necessarily, arranged next to each other. Thus, the term "detector element" can also refer to a group of detector crystals.

The detector is the functional part of the PET-scanning device, which receives and detects the emitted photons. The detector is in particular formed by the part of the PET-scanning device which comprises the detector crystals.

The detector preferably forms a closed ring, i.e. it can be closed in the circumferential direction. By forming a closed ring, the detector elements can be optimally arranged along the entire inner surface of the detector ring. If the detector forms a closed ring, it preferably as a whole has an essentially cylindrical shape. The detector advantageously has an inner surface, which is cylindrical. The cylindrical shape of the detector as a whole or of the detector's inner surface define an axial and a radial direction of the detector. An axial and a radial direction of the detector are usually also defined by the detector's inner surface, if the detector is not formed by a closed ring, but e.g. by a partially open ring. As a whole, the detector can e.g. have a circular, i.e. cylindrical, or polygonal shape.

The detector elements are preferably arranged in the form of rings, wherein each ring is composed of a plurality of detector elements, and wherein, advantageously, a plurality of such rings is arranged adjacent to each other along the axial direction of the detector. Each of the plurality of ring preferably comprises the same number of detector elements.

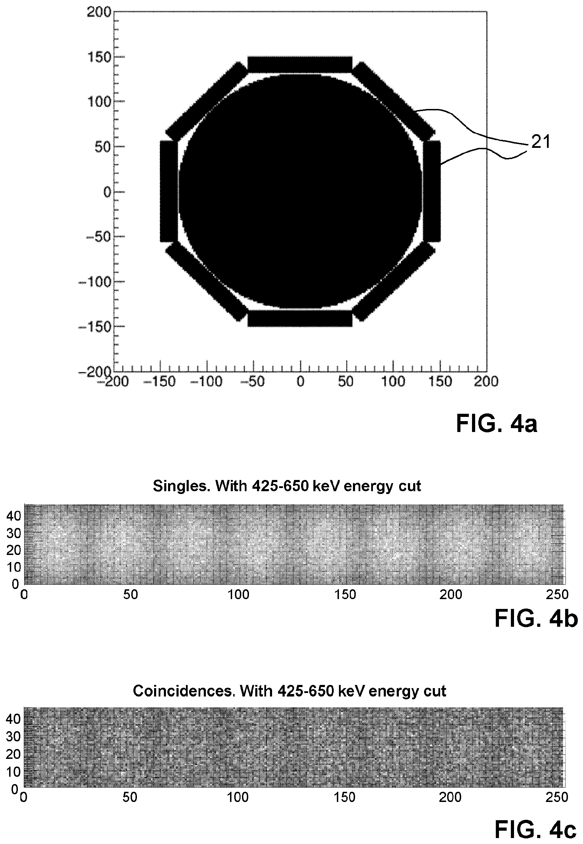

The histogram is a representation of the frequency distribution of the singles emission data detected per detector element. The representation can be in the form of a graphical visualization, e.g. to a user of the PET-scanning device. In this case, the histogram is preferably a graph, in which the y-axis designates the detector rings and the x-axis the detector elements per ring. The graph can be a three-dimensional graph, in which the z-axis designates the counts of singles as detected per detector element. The graph, however, can also be a greyscale- or color-coded graph, in which the greyscale-value or color reflects the counts of singles as detected per detector element. Respective greyscale-graphs of singles are for example shown in figures 4b, 5b, 7a and 10a. A "bin" of the histogram thus refers to the number of counts, i.e. the sum, of singles as detected by one respective detector element during a certain period of time, i.e. the acquisition time.

Usually, however, the histogram is not represented graphically to a user, but is only (or additionally) represented internally, in particular in digital form, in e.g. a processor or storage device of the PET-imaging device.

The determined relative detector element efficiencies and/or the acquired histogram data are preferably stored, in order to be further used at a later point in time, e.g. for logging and/or comparison purposes and/or to base a later determination of relative detector element efficiencies at least partly on a previous determination of relative detector element efficiencies and/or histogram acquisition.

The acquisition of the histogram of singles emission data in step a) usually involves a detection of emissions from a radioactive tracer that is present within the body of the subject to be scanned or that is present within an imaging phantom. An imaging phantom, which also referred to by simply "phantom", is a specially designed object well known to the skilled person that serves to e.g. evaluate, analyze and/or tune the performance of the PET-imaging device. For detecting the emissions from the radioactive tracer, the detector usually comprises a plurality of detector elements, which preferably form sensors and which comprise at least one detector crystal that is used to record emitted photons. The detector elements can be arranged in a circumferential or a polygonal ring, in order to be positioned around e.g. a patient's head or body for detecting PET-radiation emitted from the patient's head or body.

Thus, the histogram data can be acquired from a phantom or from a human or animal patient. An acquisition from a phantom has the advantage that a pre-known source distribution can be used that can even be optimized to the respective purpose, i.e. the determination of relative detector element efficiencies. A phantom usually has a regular source distribution and can be scanned free of motion artifacts. Furthermore, a phantom can be used that is optimized to the detector with regard to shape and size. If the histogram data are acquired from a phantom, then the phantom preferably has a length that is the same or greater than the axial length of the detector. An acquisition from a human or animal patient, on the other hand, has the advantage that self-normalization of the PET-scanning device can be applied, i.e. that the normalization is carried out based on the imaging data which are acquired anyway. No extra measurements are necessary in this case, which leads to a considerable time saving. Also, is in this case there is no time gap between the acquisition of the normalization data and the actual imaging of the patient, i.e. a drift in detector element efficiencies over time does not lead to errors in the normalization.

The PET-scanning device is preferably a brain PET-scanner, i.e. a device that is specifically adapted for the PET-imaging of the human brain. In this case, the PET-scanning device preferably comprises a detector ring which is adapted to encompass the head of an adult human patient. The size of the main opening of the detector is then advantageously optimally designed and sized to accommodate a human head therein. In other embodiments, however, the PET-scanning can of course also be a whole-body scanner having a vertical opening for receiving human patients in a horizontal position. The vertical opening is then sized such, that an adult human patient can be moved therethrough along of his/her longitudinal body axis.

For adjusting the position of the subject to be scanned relative to the detector, the subject to be scanned (which can e.g. be a human patient or a phantom) can be moved or the detector can be moved or both. Moving the detector has the advantage that the subject to be scanned has not to be bothered. If the detector is moved, it can particularly be rotated and/or translationally displaced with respect to a main supporting structure of the PET-scanning device. Therefore, in a preferred embodiment, the detector is attached to a main supporting structure in such a way that it can be translationally displaced along e.g. a guide rail of the main supporting structure. The patient can then be in a sitting or lying position during the PET-imaging and the detector can be (fine-)positioned optimally with respect to the patient without having to move the patient. In normal use of the PET-scanning device, the main supporting structure usually carries the main weight, if not the entire weight, of the detector. Thus, the main supporting structure usually serves to support and to hold the detector ring in a stable position during the scanning procedure.

The acquisition of the histogram data in step a) is usually carried out by detecting and integrating positron emissions during a certain period of time. The duration of this time period is preferably manually adjustable by an operator of the PET-scanning device. By manually adjusting the duration of this time period, the operator can optimize the data acquisition with regard to quality and time. The time period during which positron emissions are acquired and integrated in step a) can preferably be adjusted by the operator over a range of at least 1s to 1 minute. The period of time during which the histogram data are acquired is preferably less than 60s, more preferably less than 30s, and most preferably less than 10s. Due to the use of singles instead of coincidences, it is possible to achieve such short scan times for obtaining the normalization data.

In certain embodiments, the period of time during which the histogram data are acquired in step a) can automatically be adjusted by the PET-scanning device based on an evaluation of previously acquired histogram data of singles and/or on the actually acquired histogram data. For example, histogram data can be acquired in step a) until a certain total count, average count and/or median count is reached over all detector elements. Alternative or additional statistical data can be considered, in order to define the duration of acquisition. Thus, the PET-scanning device can be adapted to automatically terminate the acquisition of histogram data in step a), as soon as sufficient histogram data have been obtained with regard to a certain quality standard for determining the relative detector element efficiencies.

Good results can particularly be obtained, if the acquisition of singles is restricted to an energy window of preferably 375 - 700 keV, more preferably 400-675 keV, most preferably 425 - 650 keV.

In a particularly preferred embodiment, the high-pass filtered histogram data, in particular the determined relative detector efficiencies, are used for calibration, especially normalization, of the PET-scanning device and/or for correction of PET-data acquired by the PET-scanning device. Thus, while it is generally conceivable to use the determined relative detector efficiencies for a variety of other purposes, it is preferred to use them for the normalization of detector efficiencies of the PET-scanning device.

The method preferably additionally comprises the step of norming the histogram data to the sum or average of the singles emissions detected by all detector elements. Thus, each bin of the histogram is preferably divided by the sum over all bins of the histogram. This step of norming the histogram data is preferably carried out after the application of the high-pass filter, i.e. after step b). It is, however, also conceivable to carry out the norming between steps a) and b).

If the histogram data are acquired from a phantom in step a), then the phantom can have an outer surface which substantially deviates from the inner surface of the detector with respect to its dimensions and/or shape. It is a particular advantage of the method indicated, that relative detector efficiencies can be determined in an accurate way also in situations, where the phantom is not optimally adapted to the detector as concerns its outer dimensions and/or shape. Thus, with the method as indicated, it is not necessary to use a phantom that is specifically designed and adapted to the specific PET-scanning device. Instead, any phantom suited for PET-imaging can be used for e.g. the purpose of normalization, which can e.g. result in less costs and a more comfortable handling of the phantom.

If the detector is formed by a closed ring, in the ideal case it has a circular arrangement of detector elements, in particular of the inner faces of the detector elements, and it also has a circular inner surface, which is ideally even formed directly by the detector elements. In such an ideal case, a phantom can be provided for normalization purposes that completely fills out the entire main opening, i.e. field-of-view, of the detector.

In practice, however, such an ideal situation is usually not provided due to many reasons: Often the detector elements, i.e. the individual detector modules, have a flat inner surface, such that they together form a regular polygon with their inner faces, when arranged in a ring-like shape. Also, in order to protect the detector crystals from external influences, the detector usually comprises a cover which covers the detector elements and forms the inner surface of the detector. In many cases, the inner surface of the cover is arranged at a distance to the inner faces of the detector elements and, furthermore, does not exactly follow the inner faces of the detector elements, but e.g. has a circular or rounded shape. As a result, in practice, even if an optimized phantom is used that completely fills out the main opening of the detector and with its outer surface exactly reflects the detector's inner surface, there are still regions between the detector crystals and the phantom in which no radiating source can be placed for the normalization measurements. Due to such geometrical factors, the normalization of the PET-scanner then results in errors, when carried out according to a usual method of the state of the art. With the method as indicated, however, it is possible to accurately determine relative detector element efficiencies, even if the outer surface of the phantom substantially deviates from the inner surface of the detector of the PET-scanning device with respect to its dimensions and/or shape

The spatial high-pass filter used in step b) can basically be any type of spatial high-pass filter. The spatial high-pass filter can be applied in image-space or in Fourier-space. Preferred, however, is the use of a spatial high-pass filter in the form of a Gaussian high-pass filter. Particularly good results could by achieved in this way.

The present invention also refers to a PET-scanning device, which is preferably configured to carry out the method as indicated above, and which comprises a detector with detector elements and a control unit, the control unit being configured
- to acquire, by means of the detector, a histogram of singles emission data, wherein each bin of the histogram is associated to one of a plurality of detector elements of the detector and reflects the sum of singles emissions detected by the respective detector element during a certain period of time; and
- to apply a spatial high-pass filter to the acquired histogram of singles emission data.

The control unit, which can e.g. be arranged in a main supporting structure of the PET-scanning device, preferably has a processor, in particular a digital processor, and a storage device, i.e. a memory, in which a computer program is stored, which computer program, when executed by the processor, has the effect that the PET-scanning device performs the above-mentioned method.

Thus, a computer program, preferably stored on a storage device readable by a computer, is provided, which serves for controlling a PET-scanning device comprising a detector with detector elements, in order to determine relative detector element efficiencies, preferably according to the method as indicated, and which is preferably adapted to control a PET-scanning device as indicated. The computer program preferably comprises executable instructions to carry out the method as indicated above, and at least comprises executable instructions
- to acquire, by means of the detector, a histogram of singles emission data, wherein each bin of the histogram is associated to one of a plurality of detector elements of the detector and reflects the sum of singles emissions detected by the respective detector element during a certain period of time; and
- to apply a spatial high-pass filter to the acquired histogram of singles emission data.

Thus, the computer program carries out central parts of the method described above when executed in a processor of a PET-scanning device or in a processor being connected with a PET-scanning device. The computer program is usually realized as a computer program code element which comprises computer-implemented instructions to cause a processor to carry out a particular method. The computer program can be present in particular in the form of a computer program product on a suitable data carrier, e.g. on a CD-ROM, on a flash memory, etc., or can be provided for download via a network. It can be present in any desired form, e.g. as source code, object code or machine code.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a perspective view of an exemplary PET-scanning device in the form of a brain scanner which is adapted to carry out the inventive method, with an operator;
- Fig. 2: shows a schematic cross-sectional view through the detector of a PET-scanning device which can be inventive or according to the state of the art, with a patient's head accommodated in the detector's opening;
- Fig. 3a: shows a simulated cylindrical arrangement of sensor modules of a PET-detector within which a cylindrical phantom of uniform activity is arranged that completely fills out the detector opening;
- Fig. 3b: shows the histogram of coincidences as measured by all crystals of the detector for the case shown in Fig. 3a;
- Fig. 3c: shows the histograms of coincidences and singles summed per ring of the detector for the case shown in Fig. 3a;
- Fig. 4a: shows a simulated octagonal arrangement of sensor modules of a PET-detector within which a cylindrical phantom of uniform activity is arranged that almost completely fills out the detector opening;
- Fig. 4b: shows the histogram of singles as measured by all crystals of the detector for the case shown in Fig. 4a;
- Fig. 4c: shows the histograms of coincidences measured by all crystals of the detector for the case shown in Fig. 4a;
- Fig. 5a: shows a simulated octagonal arrangement of sensor modules of a PET-detector with a line source phantom arranged in the center of the detector opening;
- Fig. 5b: shows the histogram of singles as measured by all crystals of the detector for the case shown in Fig. 5a;
- Fig. 5c: shows the histograms of coincidences measured by all crystals of the detector for the case shown in Fig. 5a;
- Fig. 6a: shows the histograms of coincidences and singles measured by each ring of the detector for the case shown in Fig. 5a, using a line source with a length of 163.2 mm which corresponds to the axial length of the detector;
- Fig. 6b: shows the histograms of coincidences and singles measured by each ring of the detector for the case shown in Fig. 5a, using a line source with a length of 326.4 mm;
- Fig. 6c: shows the histograms of coincidences and singles measured by each ring of the detector for the case shown in Fig. 5a, using a line source with a length of 653 mm;
- Fig. 7a: shows the histogram of singles measured during 0.5s by all crystals of the detector for the case shown in Fig. 5a, using a line source with a length of 653 mm;
- Fig. 7b: shows the histogram of coincidences measured during 0.5s by all crystals of the detector for the case shown in Fig. 5a, using a line source with a length of 653 mm;
- Fig. 8a: shows a schematic cross-sectional view through the detector of another PET-scanning device which can be inventive or according to the state of the art, with four sensor modules and with a patient's head accommodated in the detector's opening;
- Fig. 8b: shows a schematic cross-sectional view through the detector of another PET- scanning device which can be inventive or according to the state of the art, with five sensor modules and with a patient's head accommodated in the detector's opening;
- Fig. 8c: shows a schematic cross-sectional view through the detector of yet another PET-scanning device which can be inventive or according to the state of the art, with eight sensor modules and with a patient's head accommodated in the detector's opening;
- Fig. 9a: shows a histogram of the efficiencies of all crystals of a detector as measured by a conventional method using a phantom, normed to the average of the bin counts;
- Fig. 9b: shows a histogram of singles as detected by each crystal of the same detector and with the same phantom as used in Fig. 9a, normed to the average of the bin counts;
- Fig. 9c: shows the reconstructed brain images of a patient obtained by a test PET-system (i, top row) and by a reference PET-system (ii, bottom row), with all corrections applied using a conventional normalization method based on the scanning of a phantom;
- Fig. 10a: shows a histogram of singles as detected by each crystal of a detector during the scan of the same patient as in Fig. 9c, normed to the average of the bin counts;
- Fig. 10b: shows the same histogram as in Fig. 10a, after applying a spatial Gaussian high-pass filter;
- Fig. 10c: shows the reconstructed brain images of the patient of Fig. 9c obtained by a test PET-system (i, top row) and by a reference PET-system (ii, bottom row), with all corrections applied based on detector efficiencies that have been determined using a high-pass filtered histogram of singles as obtained during the scan of the patient;
- Fig. 10d: compares the line profiles of the histograms of Figs. 10a and 10b;
- Fig. 11a: shows a simulated octagonal arrangement of sensor modules of a PET-detector with a line source phantom arranged decentrally in the detector opening;
- Fig. 11b: shows a histogram of singles as detected by each crystal of the detector for the case shown in Fig. 11a, normed to the average of the bin counts;
- Fig. 11c: shows the same histogram as in Fig. 11b, after applying a spatial Gaussian high-pass filter;
- Fig. 11d: compares the line profiles of the histograms of Figs. 11b and 11c;
- Fig. 12a: shows a simulated octagonal arrangement of sensor modules of a PET-detector with a decentral and non-uniform source distribution within the detector opening;
- Fig. 12b: shows a histogram of singles as detected by each crystal of the detector for the case shown in Fig. 12a, normed to the average of the bin counts;
- Fig. 12c: shows the same histogram as in Fig. 12b, after applying a spatial Gaussian high-pass filter;
- Fig. 12d: compares the line profiles of the histograms of Figs. 12b and 12c; and
- Fig. 13: shows a flow diagram for illustrating the inventive method for determining relative detector element efficiencies.

### DESCRIPTION OF PREFERRED EMBODIMENTS

Elements shown in the figures, which have the same or a similar function, but belong to different embodiments, are annotated with the same reference numerals in each case.

Figure 1 shows a PET-scanning device 1 which is adapted to carry out the inventive method. The embodiment of a PET-scanning device 1 as shown in figure 1 is a brain-scanner, i.e. a scanner which is specifically adapted for the PET-imaging of the brain of human patients.

In the embodiment as shown in figure 1, the PET-scanning device 1 comprises a detector 2 in the form of a detector ring, which has a plurality of sensors arranged along of its radial inner surface 23 which here has a circular shape. The sensors, which can also be referred to as detector crystals, are not visible in the figure. They serve to detect and measure the PET-radiation emitted in the region of the main opening of the ring-shaped detector 2.

The detector 2 is arranged between two holding arms 31 that hold the detector 2 between them. The two holding arms 31 form a part of a U-shaped portion, which is attached to a main supporting structure 3. The detector 2 is rotatable about an axis of rotation R that extends diametrically through the detector 2 and through the two holding arms 31 of the U-shaped portion. Furthermore, the detector 2 displaceable together with the U-shaped portion along an inclined displacement direction D relative to the main supporting structure 3. In order to facilitate rotation and displacement of the detector 2, one or several handles can be attached to an outer surface 24 of the detector 2. It is, however, also possible that the detector 2 is displaceable by means of a motor which can be operated by an operator O via a displacement button 35. The displacement button 35 is preferably arranged at the main supporting structure 3, as shown in Figure 1.

The direction along which the U-shaped portion with the holding arms 31 is displaceable is inclined with respect to the direction of gravity, meaning that it is neither parallel nor perpendicular to the direction of gravity. The U-shaped portion is attached to a guide rail 32 that is provided on the main supporting structure 3 and allows the U-shaped portion to be displaced along the inclined direction D as mentioned. Thus, the guide rail 32 also extends along the inclined direction.

A scanning support 4 is provided for accommodating a human patient in an inclined sitting position during the scanning procedure. Prior to the image acquisition, the patient is accommodated on the scanning support 4 with the detector 2 being in the uppermost position of the main supporting structure 3. The detector 2 is then displaced and, if necessary, rotated by the operator O into an optimal position for the image acquisition.

The scanning support 4 in the form of a chair-like seating unit comprises a base structure 41 that supports a seat base 42 and a leg support 43 for supporting the legs of the patient during the scanning procedure. Also attached to the base structure 41 is an inclined backrest 46. Attached to the backrest 46 are pivotable armrests 45 as well as a head support 44. For the image acquisition or for the acquisition of normalization data, the head of the patient rests on the head support 44 and the detector 2 is displaced and rotated such that the patient's head is arranged inside of the detector 2. Thus, the head support 44 is then likewise arranged inside of the detector 2 during the imaging or data acquisition process. In the present embodiment, the scanning support 4 comprises a base structure 41 which forms the bottom of the PET-scanning device 1 and connects the scanning support 4 to the main supporting structure 3.

The displacement direction D of the detector 2, which is defined by the longitudinal extension of the guide rails 32, approximately corresponds to the longitudinal extensions of the backrest 46 and of the head support 44. Thus, the displacement direction D of the detector 2 corresponds to the longitudinal main axis of the upper part of the body of the patient, if the patient sits on the scanning support 4 as intended and is ready for the scanning procedure. In order to prevent the patient from moving his head during the image acquisition, a head strap can be used.

A screen 33 can be attached to the main supporting structure 3, which serves to display various information of e.g. the patient's head and/or the status of the PET-scanning device 1 to the operator O.

Figure 1 also shows a possible arrangement of a control unit 34 that serves for controlling the PET-scanning device 1. The control unit 34 is here arranged within the main supporting structure 3. Data and/or energy transmission to or from the control unit 34 can be done via one or several cables and/or wirelessly. In other embodiments, the control unit 34 could also be arranged externally. The control unit 34 is preferably connected, by cable or wirelessly, to a user input and output device. The user input and output device can for example be in the form of an external personal computer or it can be a display. It is also possible for the screen 33 to be the input and output device. The screen 33 can for this purpose for example be in the form of a touch screen.

Figure 2 shows a schematic cross-sectional view through the detector 2 of a PET-scanning device 1 in the form of a brain scanner, with the head of a patient P accommodated in the detector's opening. As can be seen in the figure, the detector 2 comprises a plurality, here eight sensor modules 21, which each comprise one or more sensor crystals 22. In the present embodiment, each sensor module 21 comprises four sensor crystals 22. The sensor crystals 22, which can be regarded as detector elements, serve to record photos that are emitted from a source arranged within the main opening of the detector 2, i.e. from the head of the patient P in the present case.

As can be seen in figure 2, each of the sensor modules 21 has a flat inner surface that is formed by the sensor crystals 22. The sensor modules 21 are arranged directly adjacent to each other in such a way that a regular octagon is formed. The detector 2 comprises a cylindrical housing, i.e. a cover, which covers all sensor modules 21. The housing forms a radial inner surface 23 and a radial outer surface 24 of the detector 2. It can be easily understood from figure 2 that a phantom, even if it is optimally shaped and sized with respect to the inner surface 23, can never completely fill out the space delimited by the sensor modules 21 due to their polygonal arrangement and due to their distance to the inner surface 23. The consequences of this difference between the inner faces of the sensor modules 21 and the inner surface 23 of the detector 2 with regard to the determination of relative detector element efficiencies is explained with the help of figures 3a to 4c in the following:
Figure 3a shows a computer simulation of an almost ideal situation, with an almost circular arrangement of a large number or sensor modules 21, which allows the use of a cylindrical phantom of uniform activity distribution that fills almost the entire space, i.e. field-of-view, delimited by the sensor modules 21. The phantom length here corresponds to the axial length of the detector. For the simulation, a detector having 24 rings of sensor modules 21 was assumed. In the simulation, in order to avoid gamma scattering and attenuation, the phantom is made of air

Figure 3b shows the histogram of coincidences as measured by all crystals of the detector for the case shown in figure 3a. In the histogram of figure 3b (as well as in the ones of figures 4b, 4c, 5b, 5c, 7a, 7b, 9a, 9b, 10a, 10b, 12b and 12c), the y-axis designates the detector rings and the x-axis the detector elements, i.e. the detector crystals, per ring. The histogram has been obtained using a Monte-Carlo simulation. The measured coincidence data reflect the detector efficiencies. Since simulation data are used, a more or less uniform histogram of coincidences events can be expected. It can indeed be seen that the histogram of figure 3b has a uniform distribution of detected coincidences within an energy interval of 425 - 605 keV, i.e. no adverse effects due to geometric or other reasons are visible.

In figure 3c, the histograms of simulated coincidences and singles are shown, summed per ring of the detector for the case shown in figure 3a. It can be seen that the distribution of coincidences is more uniform than the distribution of singles, which in the state of the art justifies the use of coincidences to determine efficiencies for cylindrical PET-detectors by means of cylindrical phantoms.

Figure 4a shows another simulation of a less ideal, but more realistic situation, in which there are less sensor modules 21 that are arranged in the form of a polygon. Thus, the space of the detector that is delimited by the sensor modules 21 is non-cylindrical. As a consequence, a cylindrical phantom (as it is shown in figure 4a), even if maximized with regard to its size, does not fill out the entire space within the ring formed by the sensor modules 21. This results in a less uniform histogram of coincidences (figure 4c) which reflects the detector geometry. The (simulated) histogram of singles is likewise affected by the scanner geometry (figure 4b). To obtain more uniform histograms that better reflect the detector efficiencies, a phantom with an octagonal cross-section would be needed, which is not only more complicated to fabricate, but it is often not possible to use in practice due to a cylindrical inner surface 23 of the detector (see figure 2).

Thus, the simulations of figures 3a to 5c show that at first sight, coincidences seem to be much better suited for determining detector efficiencies: Figure 3c illustrates a clearly better result for coincidences than for singles. The result for coincidences is still slightly better or at least comparable, for the case of a realistic detector geometry with a maximized phantom as shown in figure 4a. Therefore, it does not come as a surprise that in the state of the art, direct normalization using a phantom maximized with respect to its shape and dimension, i.e. as shown in the case of figure 4a, is regarded as the golden standard for the normalization of PET-scanner devices in practice.

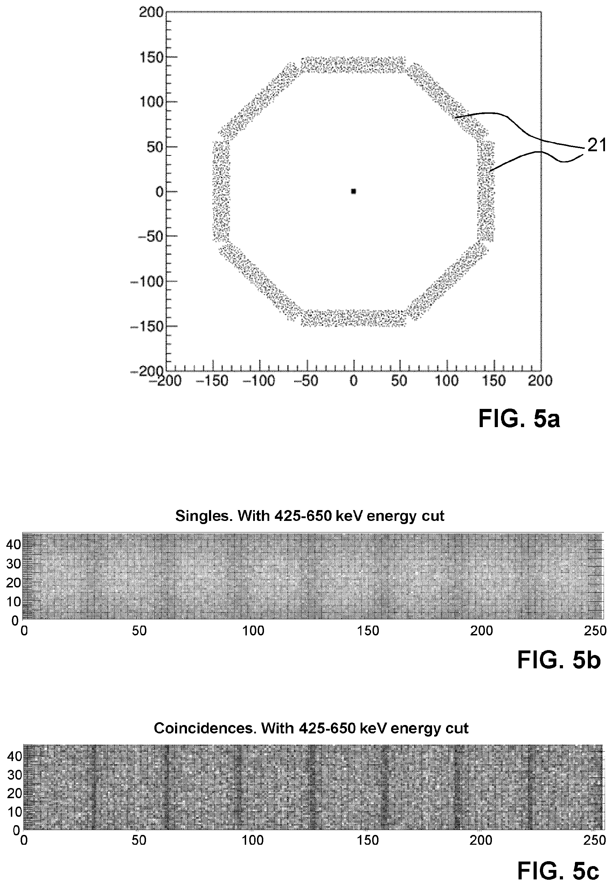

The inventors of the method as indicated in this document, however, made further considerations:
In particular, an even more extreme situation for differing from the (almost) ideal case of figure 3a than figure 4a has been simulated, which is shown in figure 5a. Figure 5a shows the same simulation as figure 4a, but with a phantom in the form of a line source.

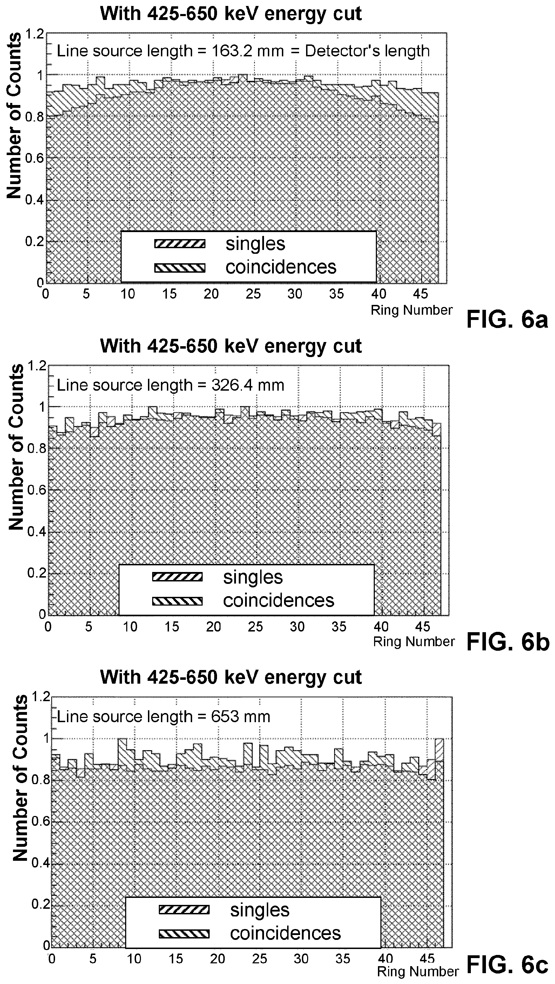

If the histograms of singles (figure 5b) and coincidences (figure 5c) obtained for the situation of figure 5a are compared, it can be seen that the coincidences histogram is severely affected by both the geometry of the detector and the geometry of the phantom. However, as it turns out, the histograms of singles is affected by the geometry of the detector, but less by the geometry of the phantom. In the histogram of Figure 5b, the geometry of the detector is evident not only in the form of regular vertical stripes due to the octagonal arrangement of the sensor modules 21, but also in the form of a slight decrease in the emission counts toward each axial end of the detector, i.e., toward the detector rings with the lowest and highest indices on the y-axis. This effect is examined in more detail in figures 6a to 6c:
Figure 6a reflects the situation for the case of figure 5a, with a line that has the same length as the detector. A pronounced decrease of counts towards the axial ends of the detector is clearly visible for singles and also, but to a much lesser extent, for coincidences. If the length of the line source is increased, this effect becomes less and less pronounced (figures 6b and 6c). What becomes more pronounced, however, is the noise in the coincidences data, i.e. the contamination of the histogram with random events, which does not occur, or to a much lesser extent, in the singles data. Thus, as it turns out from these simulations of figures 5a to 6c, in general, the best results with regard to the determination of relative detector element efficiencies can surprisingly be obtained based on a histogram acquisition of singles and with a source that has a length which is the same or greater than the axial length of the detector.

From the simulations of figures 5a to 6c, the inventive idea of using single events to determine detector efficiencies for non-cylindrical detectors and non-ideal source distributions can be understood. By the application of a spatial high-pass filter to the obtained histogram, it is possible to remove or at least greatly reduce the effects due to the geometry of the detector with respect to the source distribution (i.e. the vertical stripes in figure 5b). Thus, with the inventive method, relative detector element efficiencies can be determined in practical situations even with the help of a phantom in the form of a line source. The use of a phantom having rather the shape of a line source than of a cylindrical phantom with maximized diameter has several advantages: The phantom is smaller and lighter and therefore easy to handle. Also, the gammas emitted from the line source are not attenuated and, therefore, not scattered, such that it is not or to a much smaller degree necessary to correct the acquired data for these events, which makes data processing faster and easier. Additionally, with the method as indicated, it is not needed to precisely center the phantom in the field-of-view of the detector. The phantom also does not need to be a line source. With the method as indicated, any phantom will work, and it is even possible to use patient data to determine relative detector element efficiencies (self-normalization).

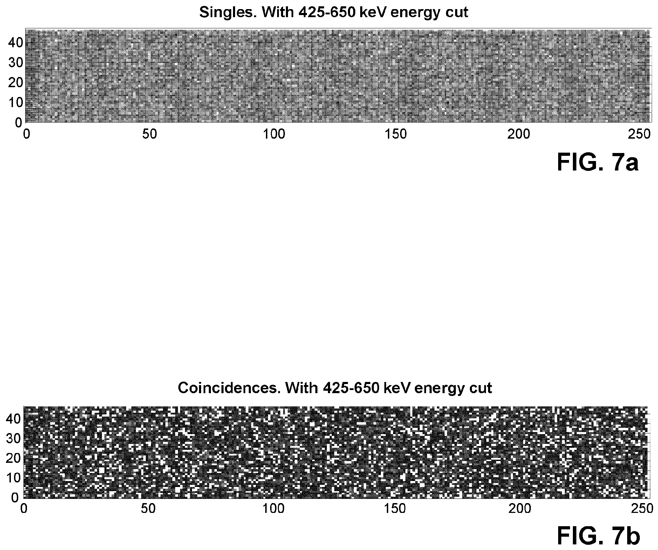

Figures 7a and 7b show simulated histograms for visualizing the further advantage of the singles acquisition as compared to the coincidences acquisition with regard to the necessary acquisition time: A 0.5-second-long data acquisition has been simulated for a 653-mm-long line source (i.e. the cross-sectional situation as shown in figure 5a). In the histograms of figures 7a and 7b, "white" pixels of the coincidence histogram indicate that there was no coincidence data in the corresponding detector (due to a very short acquisition). The single histogram does not have any white pixels. Thus, it is another advantage of using singles over coincidences, that the necessary data can be collected in a very short time.

Since with a singles acquisition and subsequent high-pass filtering, the relative detector element efficiencies can be determined within a short time and based on non-uniform source distributions, it becomes possible to use patient data to determine relative detector element efficiencies. For this purpose, unlike in normalization methods of the state of the art, the patient does not necessarily need to be moved during the data acquisition.

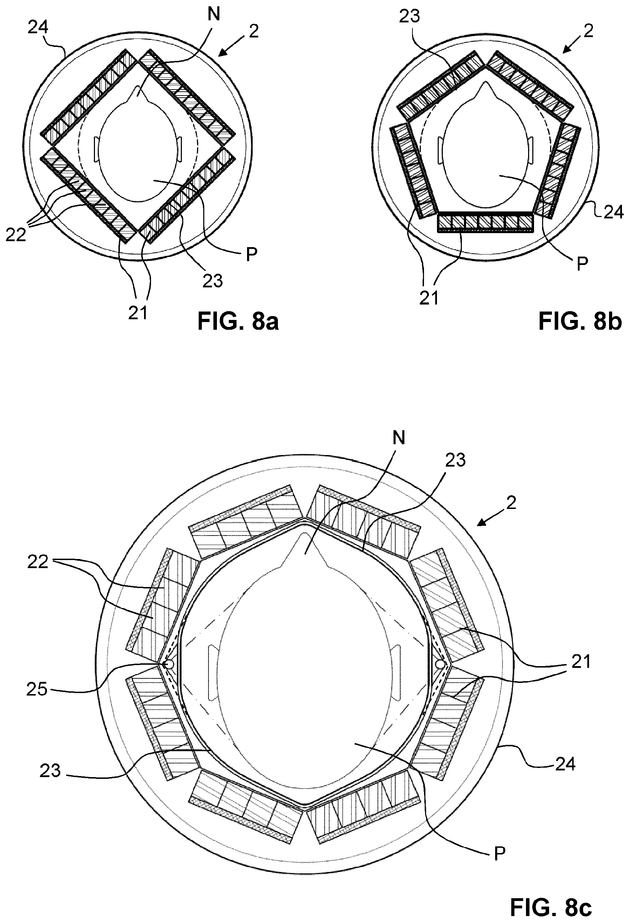

Figures 8a to 8c show further possible embodiments of a detector 2 with four sensor modules 21 arranged in a square (figure 8a), with five sensor modules 21 arranged in a pentagon (figure 8b) and with eight sensor modules 21 arranged in an octagon (figure 8c).

As can be seen from figures 8a to 8c, the space to accommodate the patient's nose N is particularly large, if the arrangement of the sensor modules 21 results in a polygon with less corners. On the other hand, if the polygon has more corners, the sensor modules 21 become smaller and are directed with a larger section of their detector surface towards the center of the detector's opening.

The shape of the inner surface 23 can correspond to the polygonal shape that is formed by the sensor modules 21, in order to maximize the detector's opening. It is, however, also possible that the inner surface 23 describes a curvature that e.g. corresponds to the incircle of the polygon at least in the areas of some of the corners, as it is illustrated in figures 8a and 8b by dashed lines. In this way, the inner surface 23 has a smooth and rounded design. However, for direct normalization, it is not possible anymore to fill out the space in the respective corners formed by the sensor modules 21 with a phantom.

Figure 8c shows a particularly preferred embodiment of a detector 2, in which the sensor modules 21 are arranged in the form of an octagon. The inner surface 23 reflects the shape of the octagon particularly in the corners of the nose N and of the back of the head, in order to maximize the detector's opening in these regions. The space of the corners near the ears is here covered by a flattened part of the inner surface 23, in order to accommodate a laser positioning device 25 in each case. In the corners between the ones near the nose N, the back of the head and the ears, the inner surface 23 describes a curvature that corresponds to the incircle of the octagon. Thus, the inner surface 23 has a smooth, rounded design along of its entire circumference, while at the same time offering a large opening to accommodate the head of the patient P. While such a non-circular design of the inner surface 23 has considerable advantages with regard to the adaptation of the detector to the a patient's head, it is not suited for placing a cylindrical phantom with maximal diameter within the detector's opening in such a way, that it completely fills out the space delimited by the sensor modules 21. With the method as proposed in this document, however, it is nevertheless possible to achieve an accurate and fast normalization of a PET-scanning device even in such situations.

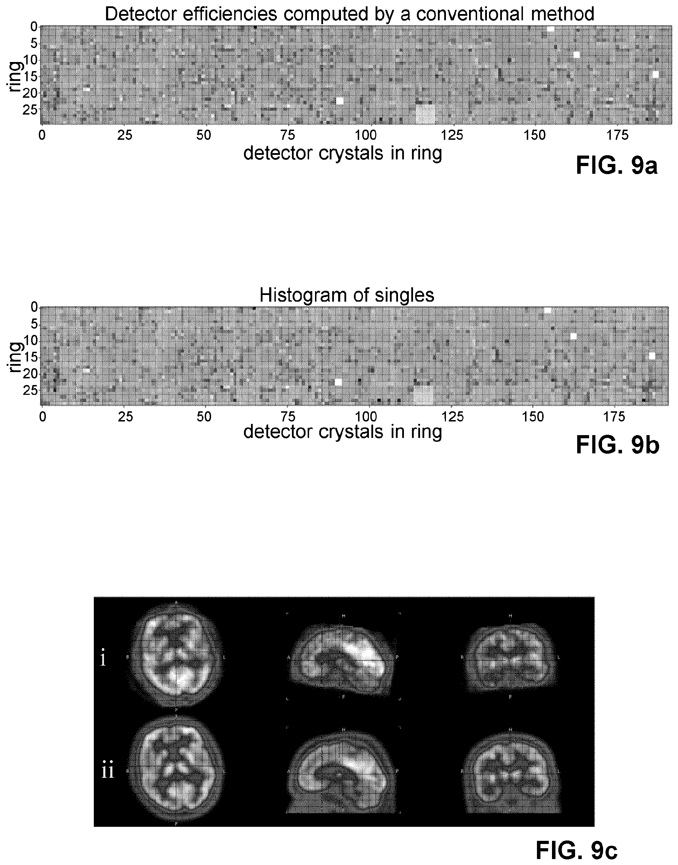

Figure 9a shows a detector efficiencies histogram obtained by a conventional method according to the state of the art, normed to the average of the bin counts. The conventional method involves scanning a cylindrical phantom with maximized diameter centered in the PET-scanner's field-of-view. The standard settings for the scan were the following: 1) the phantom is uniformly filled with a source having a low and known amount of activity. The low activity eliminates spurious random coincidences; 2) The phantom axially has the same length as the field-of-view of the PET-scanner, which effects that all possible pairs of detector crystals should experience the same coincidences rate; 3) the phantom is scanned for a sufficiently long time, in order to collect a statistically significant amount of coincidences for the normalization procedure; 4) The acquired phantom sinogram is corrected for activity decay, attenuation and scatter effects and for the dead-time of the PET-scanner before calculating the normalization factors. Figure 9b shows the histogram of singles events of the same cylindrical phantom normed to the average of the bins counts. As one can see, both histograms are almost identical and therefore both can be used to determine relative detector efficiencies for the normalization procedure. The slight differences in the histograms can be explained by imperfections of the random and scatter corrections. In the case of this optimally adapted phantom there is no need to apply a high-pass filter to the singles histogram as there are no low frequency effects that need to be removed.

Figure 9c, i (top row) shows three different view of a final reconstructed brain of a patient obtained by a test PET-system with all data corrections applied including the normalization via the scan of a phantom optimized as indicated above with respect of figures 9a and 9b. Figure 9c, ii (bottom row) shows the brain images of the same patient obtained by a reference PET system and co-registered with the test PET-brain images for comparison. One can clearly see that the activity distributions in the test and reference brain images are different.

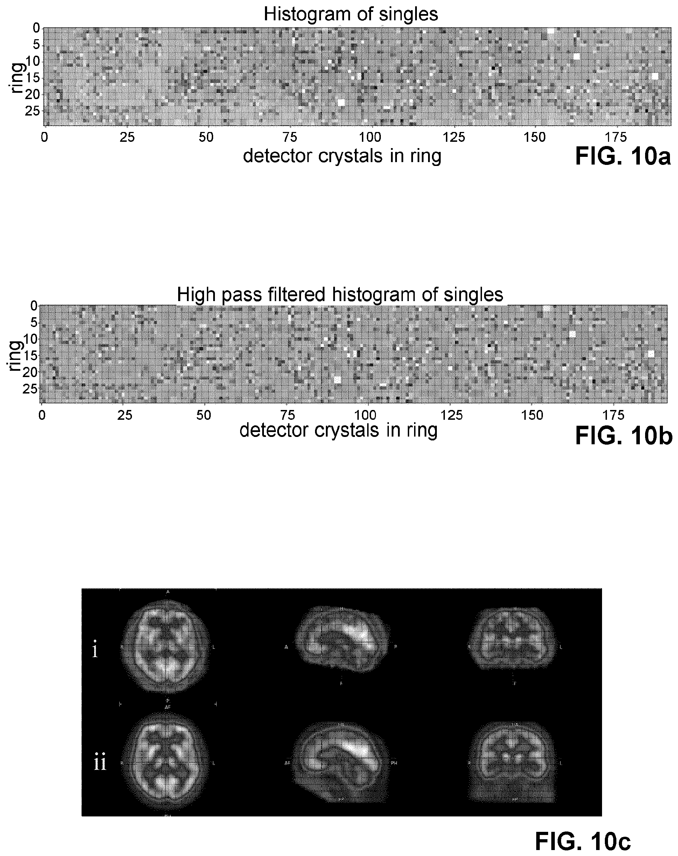

Figures 10a to 10c visualize the PET-imaging of the same patient as in figure 9c with the application of a self-normalization based on the inventive method:
Figure 10a shows a histogram of single events collected during the scan of the same patient head as in figure 9c, normed to the average of the bin counts. Figure 10b shows the same histogram after applying a Gaussian spatial high-pass filter (the filter implemented as subtracted Gaussian filter, with sigma equal to 10x estimated histogram noise sigma). The high frequencies pattern is similar to the one of figure 9b, but with small differences due to the fact that the scan of the phantom (figure 9b) was done a long time before the scan of the patient (figure 10b) and that, therefore, the detector efficiencies were altered between the two scans.

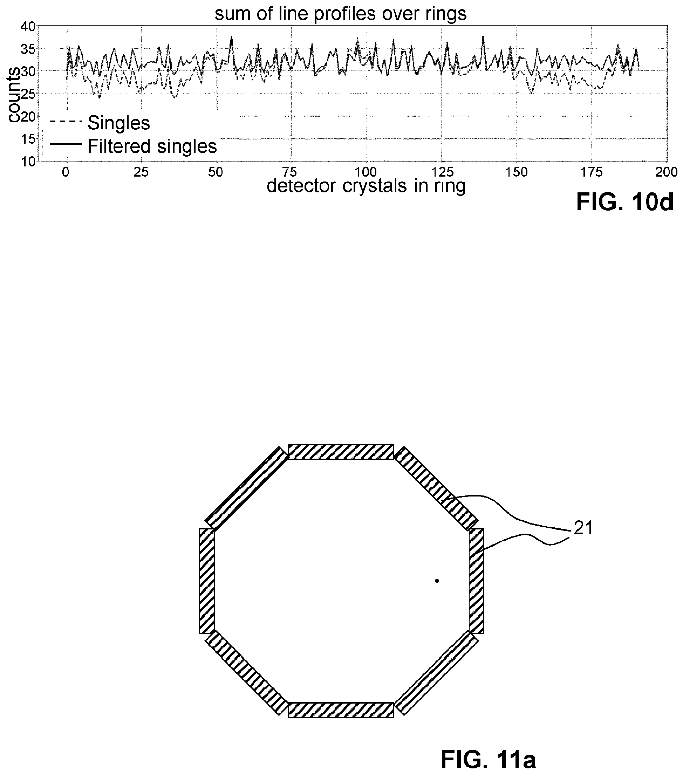

Figure 10d compares the line profiles per detector ring of the histograms of figures 10a and 10b before and after applying the spatial high-pass filter. The low frequency component of the pre-filtered line profile occurs due to a non-uniform distribution of the activity in the field-of-view, in particular because the patient head was not centered in the scanner's field-of-view. After applying the high-pass filter, the low frequency component is removed, while the high frequencies are preserved.

Figure 10c, shows reconstructed brain images obtained with the test PET-system (i, top row) and with the reference PET-system (ii, bottom row), with all corrections applied including the normalization based on the relative detector efficiencies of the histogram of figure 10b. Now the activity distribution of the test brain images (i, top row) matches the activity distribution of the reference brain image (ii, bottom row). This example of figures 10a to 10c shows that the inventive method improves the image quality compared to the conventional method (figures 9a to 9c).

Applied during a daily routine scan or equivalent, the conventional method is more complex than the inventive method and often suffers from errors coming from necessary additional data corrections. Also, the conventional method requires a person who performs the scan with the optimized phantom. The self- normalization using the inventive method, however, can account for short-term fluctuations in detector efficiencies, which can impair the results of the conventional method if occurring between the phantom scan and the patient scan.

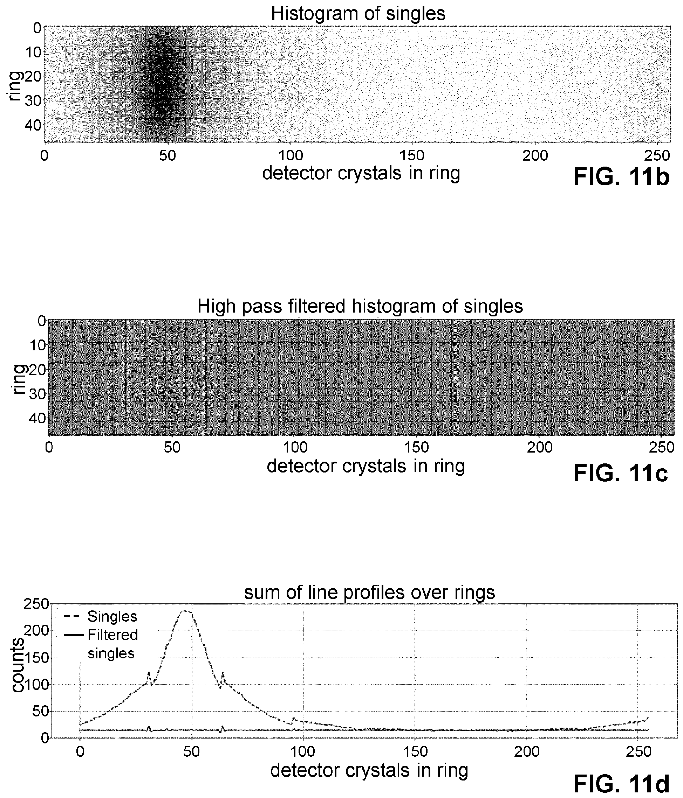

In order to show that the inventive method works in even more extreme conditions, a PET-scanner with uniform detector efficiencies was simulated (figure 11a). The detector efficiencies were calculated using singles data obtained from a simulated line source placed decentrally within the field-of-view, as shown in figure 11a. The length of the line source is equal to the length of the axial field-of-view of the scanner. Figure 11b shows the acquired singles histogram. Figure 11c shows the same histogram after applying a spatial high-pass filter (specifically a Gaussian high pass filter with sigma = 10x histogram estimated noise sigma). After filtering, the histogram is uniform which is also shown via a line profile comparison in figure 11d. The error in the calculated detector efficiencies is ~5%.

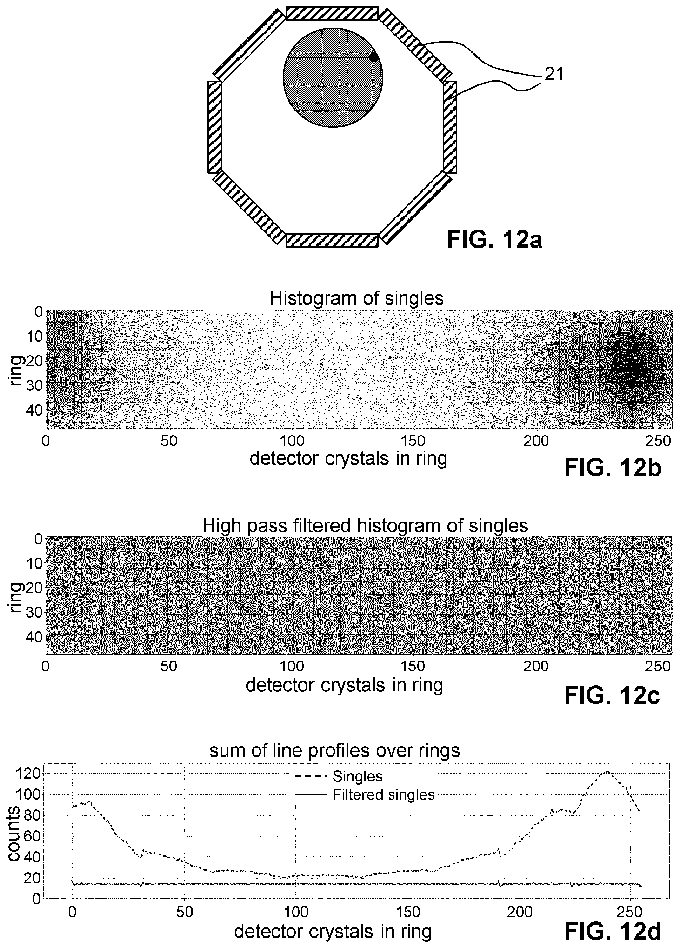

Figure 12a shows another simulation of a PET-scanner with uniform detector efficiencies and a non-uniform activity distribution in the field-of-view. Figure 12b shows the singles histogram acquired during a time period of 1 second. Figure 12c shows the same histogram after applying a high-pass filter (specifically a Gaussian high-pass filter with sigma = 10x histogram estimated noise sigma). Figure 12d compares the line profiles before and after filtering. In this case, the error in the calculated detector efficiencies is ~4%. The error of the inventive method can be reduced by increasing the acquisition time or/and by placing the source closer to the field-of-view center or/and by applying a narrower energy window (around the energy of interest) to the singles. The results above are obtained with the energy window of 425 - 650 keV. The method results in larger errors, if applied to a point source placed at the edge of the field-of-view. Therefore, the use of a line source is preferred over the use of a point source, and a more central placement of the source is preferred over a more decentral placement of the source.

The inventive method works with a very broad set of sources, such as for example: 1. A spiral source. The spiral source can for example be in the form of a cylindrical spiral spring which spins around the axial axis of the detector and extends through the entire axial field-of-view. The diameter of such a cylindrical spring source can be equal to the diameter of the transaxial field-of- view. The use of such a spiral source eliminates the use of attenuation and scatter corrections, because the probability of photons to be attenuated or scattered within the thin tube of the source is very low. 2. A line source which is preferably axially longer than the scanner's FOV and well centered in the scanner's FOV to make sure that all detectors are exposed to the same singles count rate. In this way, a particularly fast acquisition of singles can be carried out. 3. Using the natural scintillator crystals radioactive background to obtain singles rate histogram and derive detector efficiencies. This method has advantages as it uses no phantom whatsoever. However, it can be used only with PET-scanners that comprise scintillating crystals which are naturally radioactive (e.g. LSO, LYSO). 4. The patient. Thus, the patient data are in this case directly used to determine relative detector element efficiencies based on the inventive method. Using this method, a drift of detector efficiencies has no influences on the final PET-images. In addition, this method does not require additional scans, minimizing overall scan time and operator effort.

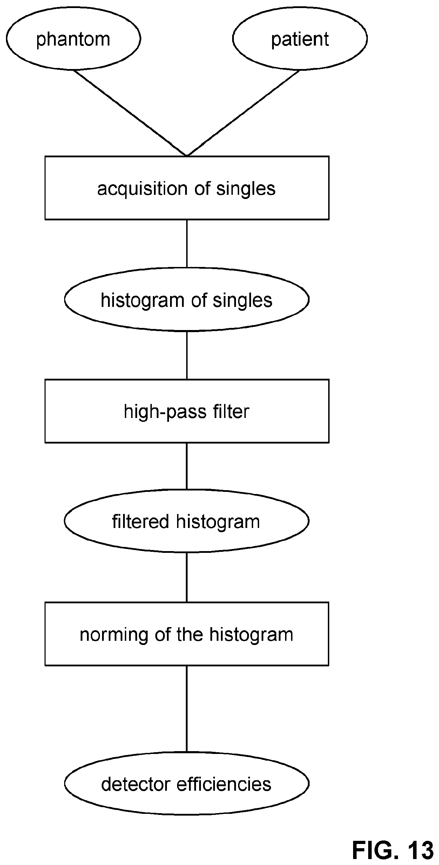

To sum up, figure 13 visualizes the individual steps of the inventive method in a flow diagram: In a first step, singles emission data are acquired from a phantom or a patient by means of a detector of a PET-scanning device. The acquired singles data are represented, e.g. internally by a control unit, in the form of a histogram. In the histogram, each bin is associated to one of the detector elements of the detector and, thus, reflects the sum of singles emissions detected by the respective detector element during a certain period of time, i.e. the acquisition time. The detector elements can particularly be formed by the individual detector crystals. In the next step, a spatial high-pass filter is applied to the acquired histogram of singles, in order to yield a filtered histogram. The filtered histogram data can then be normed to the sum or average of the singles emissions detected by all detector elements. In this way, the relative detector efficiencies of the PET-scanning device can be determined in a particularly fast and reliable manner. The obtained relative detector efficiencies can then be used for calibration, in particular normalization, of the PET-scanning device and/or for correction of PET-data acquired by the PET-scanning device.

### LIST OF REFERENCE SIGNS

| | | | |
|---|---|---|---|
| 1 | PET-scanning device | 4 | Scanning support |
| 2 | Detector | 41 | Base structure |
| 21 | Sensor module | 42 | Seat base |
| 22 | Sensor crystal | 43 | Leg support |
| 23 | Inner surface | 44 | Head support |
| 24 | Outer surface | 45 | Armrest |
| 25 | Laser positioning device | 46 | Backrest |
| 3 | Main supporting structure | R | Axis of rotation |
| 31 | Holding arm | D | Displacement direction |
| 32 | Guide rail | | |
| 33 | Screen | O | Operator |
| 34 | Control unit | P | Patient |
| 35 | Displacement button | N | Nose |

## Claims

1. A method for determining relative detector element (22) efficiencies in a positron emission tomography (PET)-scanning device (1), comprising the steps of:
a) acquiring a histogram of singles emission data by means of a detector (2) of the PET-scanning device (1), wherein each bin of the histogram is associated to one of a plurality of detector elements (22) of the detector (2) and reflects the sum of singles emissions detected by the respective detector element (22) during a certain period of time; and
**characterised in**:
b) applying a spatial high-pass filter to the acquired histogram of singles emission data.

2. The method according to claim 1, wherein the high-pass filtered histogram data are used for calibration of the PET-scanning device (1) and/or for correction of PET-data acquired by the PET-scanning device (1).

3. The method according to claim 1 or 2, additionally comprising the step of norming the histogram data to the sum or average of the singles emissions detected by all detector elements.

4. The method as claimed in any of the preceding claims, wherein each detector (22) element is formed by a single crystal of the detector (2) of the PET-scanning device (1).

5. The method as claimed in any of the preceding claims, wherein the histogram data are acquired from a human or animal patient.

6. The method as claimed in any of claims 1 to 4, wherein the histogram data are acquired from a phantom.

7. The method as claimed in claim 6, wherein the outer surface of the phantom substantially deviates from the inner surface (23) of the detector (2) of the PET-scanning device (1) with respect to its dimensions and/or shape.

8. The method as claimed in any of the preceding claims, wherein the detector (2) of the PET-scanning device (1) has a non-circular inner surface (23).

9. The method as claimed in any of the preceding claims, wherein the period of time during which the histogram data are acquired is less than 60s, preferably less than 30s, and most preferably less than 10s.

10. The method as claimed in any of the preceding claims, wherein the spatial high-pass filter is a Gaussian high-pass filter.

11. A PET-scanning device (1), which is preferably configured to carry out the method as claimed in one of the preceding claims, and which comprises a detector (2) with detector elements (22) and a control unit (34), the control unit (34) being configured
- to acquire, by means of the detector (2), a histogram of singles emission data, wherein each bin of the histogram is associated to one of a plurality of detector elements (22) of the detector (2) and reflects the sum of singles emissions detected by the respective detector element (22) during a certain period of time; and
**characterised in that** the control unit (34) is further configured:
- to apply a spatial high-pass filter to the acquired histogram of singles emission data.

12. A computer program for controlling a PET-scanning device (1) having a detector (2) with detector elements (22), and a control unit (34), wherein the computer program comprises executable instructions which, when executed by a processor of the controller or by a processor connected with the PET-scanning device, allow:
- to acquire, by means of the detector (2), a histogram of singles emission data, wherein each bin of the histogram is associated to one of a plurality of detector elements (22) of the detector (2) and reflects the sum of singles emissions detected by the respective detector element (22) during a certain period of time; and
**characterised in that** the instructions further allow:
- to apply, by means of the control unit (34), a spatial high-pass filter to the acquired histogram of singles emission data.

## Patentansprüche

1. Ein Verfahren zur Bestimmung der relativen Effizienzen von Detektorelementen (22) in einer Positronen-Emissions-Tomographie (PET)-Scanvorrichtung (1), aufweisend die folgenden Schritte:
a) Erfassen eines Histogramms von Einzelemissionsdaten mittels eines Detektors (2) der PET-Scanvorrichtung (1), wobei jeder Bin des Histogramms einem von mehreren Detektorelementen (22) des Detektors (2) zugeordnet ist und die Summe der von dem jeweiligen Detektorelement (22) während eines bestimmten Zeitraums erfassten Einzelemissionen widerspiegelt; und
**gekennzeichnet durch**
b) Anwenden eines räumlichen Hochpassfilters auf das erfasste Histogramm der Einzelemissionsdaten.

2. Das Verfahren nach Anspruch 1, wobei die hochpassgefilterten Histogrammdaten zur Kalibrierung der PET-Scanvorrichtung (1) und/oder zur Korrektur von durch die PET-Scanvorrichtung (1) erfassten PET-Daten verwendet werden.

3. Das Verfahren nach Anspruch 1 oder 2, welches zusätzlich den Schritt aufweist, dass die Histogrammdaten auf die Summe oder den Mittelwert der von allen Detektorelementen erfassten Einzelemissionen normiert werden.

4. Das Verfahren nach einem der vorstehenden Ansprüche, wobei jedes Detektorelement (22) aus einem einzelnen Kristall des Detektors (2) der PET-Scanvorrichtung (1) gebildet ist.

5. Das Verfahren nach einem der vorstehenden Ansprüche, wobei die Histogrammdaten von einem menschlichen oder tierischen Patienten erfasst werden.

6. Das Verfahren nach einem der Ansprüche 1 bis 4, wobei die Histogrammdaten von einem Phantom erfasst werden.

7. Das Verfahren nach Anspruch 6, wobei die äussere Oberfläche des Phantoms hinsichtlich ihrer Abmessungen und/oder Form erheblich von der inneren Oberfläche (23) des Detektors (2) der PET-Scanvorrichtung (1) abweicht.

8. Das Verfahren nach einem der vorstehenden Ansprüche, wobei der Detektor (2) der PET-Scanvorrichtung (1) eine nicht kreisförmige innere Oberfläche (23) aufweist.

9. Das Verfahren nach einem der vorstehenden Ansprüche, wobei der Zeitraum, während dessen die Histogrammdaten erfasst werden, weniger als 60s, bevorzugt weniger als 30s und am bevorzugtesten weniger als 10s beträgt.

10. Das Verfahren nach einem der vorstehenden Ansprüche, wobei das räumliche Hochpassfilter ein Gausssches Hochpassfilter ist.

11. Eine PET-Scanvorrichtung (1), welche vorzugsweise dazu konfiguriert ist, das Verfahren nach einem der vorstehenden Ansprüche durchzuführen, und welche einen Detektor (2) mit Detektorelementen (22) sowie eine Steuereinheit (34) aufweist, wobei die Steuereinheit (34) dazu konfiguriert ist
- mittels des Detektors (2) ein Histogramm von Einzelemissionsdaten zu erfassen, wobei jeder Bin des Histogramms einem von mehreren Detektorelementen (22) des Detektors (2) zugeordnet ist und die Summe der von dem jeweiligen Detektorelement (22) während eines bestimmten Zeitraums erfassten Einzelemissionen widerspiegelt; und
**dadurch gekennzeichnet, dass** die Steuereinheit (34) ausserdem dazu konfiguriert ist
- ein räumliches Hochpassfilter auf das erfasste Histogramm der Einzelemissionsdaten anzuwenden.

12. Ein Computerprogramm zur Steuerung einer PET-Scanvorrichtung (1), welche einen Detektor (2) mit Detektorelementen (22) sowie eine Steuereinheit (34) hat, wobei das Computerprogramm ausführbare Instruktionen aufweist, welche bei Ausführung durch einen Prozessor der Steuerung oder durch einen mit der PET-Scanvorrichtung verbundenen Prozessor erlauben
- mittels des Detektors (2) ein Histogramms von Einzelemissionsdaten zu erfassen, wobei jeder Bin des Histogramms einem von mehreren Detektorelementen (22) des Detektors (2) zugeordnet ist und die Summe der von dem jeweiligen Detektorelement (22) während eines bestimmten Zeitraums erfassten Einzelemissionen widerspiegelt; und
**dadurch gekennzeichnet, dass** die Instruktionen ausserdem erlauben
- mittels des Detektors (2) einen räumlichen Hochpassfilter auf das erfasste Histogramm der Einzelemissionsdaten anzuwenden.

## Revendications

1. Un procédé de détermination des efficacités relatives des éléments détecteurs (22) dans un dispositif de tomographie par émission de positons (TEP) (1), comprenant les étapes suivantes :
a) acquisition d'un histogramme de données d'émission individuelles au moyen d'un détecteur (2) du dispositif de tomographie par émission de positons (1), chaque case de l'histogramme étant associé à l'un des éléments détecteurs (22) du détecteur (2) et reflétant la somme des émissions individuelles détectées par l'élément détecteur (22) respectif pendant une certaine période de temps ; et **caractérisé en ce que**
b) application d'un filtre passe-haut spatial à l'histogramme acquis des données d'émission individuelles.

2. Le procédé selon la revendication 1, dans lequel les données d'histogramme filtrées passe-haut sont utilisées pour l'étalonnage du dispositif de balayage PET (1) et/ou pour la correction des données PET acquises par le dispositif de balayage PET (1).

3. Le procédé selon la revendication 1 ou 2, comprenant en outre l'étape de normalisation des données de l'histogramme à la somme ou à la moyenne des émissions individuelles détectées par tous les éléments du détecteur.

4. Le procédé tel que revendiqué dans l'une quelconque des revendications précédentes, dans lequel chaque élément détecteur (22) est formé par un cristal individuel du détecteur (2) du dispositif de balayage PET (1).

5. Le procédé selon l'une quelconque des revendications précédentes, dans lequel les données d'histogramme sont acquises à partir d'un patient humain ou animal.

6. Le procédé selon l'une quelconque des revendications 1 à 4, dans lequel les données d'histogramme sont acquises à partir d'un fantôme.

7. Le procédé selon la revendication 6, dans lequel la surface extérieure du fantôme s'écarte sensiblement de la surface intérieure (23) du détecteur (2) du dispositif de balayage PET (1) en ce qui concerne ses dimensions et/ou sa forme.

8. Le procédé tel que revendiqué dans l'une quelconque des revendications précédentes, dans lequel le détecteur (2) du dispositif de balayage PET (1) a une surface intérieure non circulaire (23).

9. Le procédé selon l'une quelconque des revendications précédentes, dans lequel la période de temps pendant laquelle les données d'histogramme sont acquises est inférieure à 60 s, de préférence inférieure à 30 s et très particulièrement inférieure à 10 s.

10. Le procédé selon l'une quelconque des revendications précédentes, dans lequel le filtre passe-haut spatial est un filtre passe-haut gaussien.

11. Un dispositif de tomographie par émission de positons (TEP) (1), de préférence configuré pour mettre en œuvre le procédé selon l'une des revendications précédentes, et comprenant un détecteur (2) doté d'éléments détecteurs (22) et une unité de commande (34), l'unité de commande (34) étant configurée pour:
a. acquérir, au moyen du détecteur (2), un histogramme de données d'émission individuelles, chaque case de l'histogramme étant associée à un parmi une pluralité d' éléments détecteurs (22) du détecteur (2) et reflétant la somme des émissions individuelles détectées par l'élément détecteur (22) respectif pendant une certaine période de temps ; et
**caractérisé en ce que** l'unité de commande (34) est en outre configurée pour:
b. appliquer un filtre passe-haut spatial à l'histogramme acquis des données d'émission individuelles.

12. Un programme informatique destiné à commander un appareil de tomographie par émission de positons (TEP) (1) comportant un détecteur (2) avec des éléments détecteurs (22), et une unité de commande (34), dans lequel le programme informatique comprend des instructions exécutables, qui, lorsqu'elles sont exécutées par un processeur de l'unité de commande ou un processeur relié au dispositif de balayage TEP, permettent de:
a. acquérir, au moyen du détecteur (2), un histogramme de données d'émission individuelles, chaque case de l'histogramme étant associée à un parmi une pluralité d'éléments détecteurs (22) du détecteur (2) et reflétant la somme des émissions individuelles détectées par l'élément détecteur (22) respectif pendant une certaine période de temps ; et
**caractérisé en ce que** les instructions permettent en outre de:
b. appliquer, au moyen de l'unité de contrôle (34), un filtre passe-haut spatial à l'histogramme acquis des données d'émission individuelles.
